# EUROPÄISCHE PATENTSCHRIFT

(11) **EP 0 730 583 B1**
(45) Veröffentlichungstag und Bekanntmachung des Hinweises auf die Patenterteilung: **28.03.2001**
(21) Anmeldenummer: 95901378.0
(22) Anmeldetag: 14.11.1994
(51) Int. Cl.: C07D 239/52, C07D 239/34, C07D 405/12, A01N 43/54

(54) **3-METHOXY-2-PHENYL-ACRYLSÄUREMETHYLESTER**
3-METHOXY-PHENYL-ACRYLIC ACID METHYL ESTERS
METHYLESTERS D'ACIDE 3-METHOXY-2-PHENYL-ACRYLIQUE

(30) Priorität: 25.11.1993 DE 4340181
(43) Veröffentlichungstag der Anmeldung: 11.09.1996
(73) Patentinhaber: BAYER AG, 51368 Leverkusen (DE)
(72) Erfinder: GAYER, Herbert, D-40789 Monheim (DE); GERDES, Peter, D-52080 Aachen (DE); SCHALLNER, Otto, D-40789 Monheim (DE); DUTZMANN, Stefan, D-40721 Hilden (DE); DEHNE, Heinz-Wilhelm, D-53125 Bonn (DE); HÄNSSLER, Gerd, D-51381 Leverkusen (DE)
(86) Internationale Anmeldenummer: EP9403773
(87) Internationale Veröffentlichungsnummer: WO9514674

(56) Entgegenhaltungen:
- EP-A- 0 382 375
- EP-A- 0 383 117
- EP-A- 0 391 451

## Beschreibung

Die Erfindung betrifft 3-Methoxy-2-phenyl-acrylsäuremethylester, ein Verfahren zu ihrer Herstellung und ihre Verwendung als Schädlingsbekämpfungsmittel.

Es ist bekannt, daß bestimmte 3-Methoxy-acrylsäuremethylester, wie beispielsweise die Verbindung 3-Methoxy-2-(6-phenyl-2-pyridylthio)-acrylsäuremethylester oder die Verbindung 3-Methoxy-2-[N-(6-phenyl-2-pyridyl)-N-methyl-amino]-acrylsäuremethylester oder die Verbindung 3-Methoxy-2-[5-(4-chlorphenyl)-3-pyridyloxy]-acrylsäuremethylester fungizide Eigenschaften besitzen (vergl. z.B. EP 383 117). Weiterhin sind aus den Dokumenten EP-A-0 391 451 und EP-A-0 382 375 3-Methoxy-acrylsäuremethylester bekannt.

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue 3-Methoxy-2-phenyl-acrylsäuremethylester der allgemeinen Formel (I), 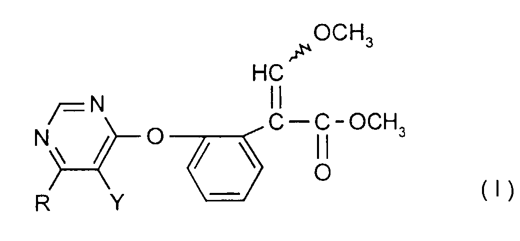 in welcher
- Y: für Fluor oder Chlor steht,
- R: für Fluor, Chlor, Brom oder für einen Rest der Formel Ar-Z- steht, wobei
- Ar: für jeweils gegebenenfalls einfach bis dreifach, gleich oder verschieden substituiertes Phenyl oder Naphthyl oder für jeweils gegebenenfalls einfach bis dreifach, gleich oder verschieden substituiertes und/oder benzoannelliertes Furanyl, Thienyl, Pyrrolyl, Oxazolyl, Thiazolyl, Isoxazolyl, Isothiazolyl, Pyrazolyl, Imidazolyl, Oxadiazolyl, Thiadiazolyl, Triazolyl, Pyridyl, Pyridazinyl, Pyrimidinyl, Pyrazinyl oder Triazinyl steht, wobei als Substituenten jeweils infrage kommen:
Fluor, Chlor, Brom, Hydroxy, Cyano, Nitro, Amino, Formyl, Carbamoyl, Thiocarbamoyl, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Allyl, Butenyl, Allyloxy, Butenyloxy, Trifluormethyl, Trifluormethoxy, Trifluormethylthio, Trifluormethylsulfinyl, Trifluormethylsulfonyl, Dimethylamino, Diethylamino, Acetyl, Acetoxy, Methylsulfonyloxy, Ethylsulfonyloxy, Methoxycarbonyl, Ethoxycarbonyl, Hydroximinomethyl, Hydroximinoethyl, Methoximinomethyl, Methoximinoethyl, Ethoximinomethyl, Ethoximinoethyl, Propan-1,3-diyl, Butan-1,4-diyl, Dioxyethylen, Dioxyethylen, Dioxypropylen, Difluordioxymethylen, Tetrafluordioxyethylen, Cyclopropyl, Cyclopentyl, Cyclohexyl, 1-Pyrrolidinyl, 1-Piperidinyl, 1-Perhydroazepinyl, 4-Morpholinyl oder jeweils gegebenenfalls ein- bis dreifach, gleich oder verschieden durch Fluor, Chlor, Brom, Methyl, Ethyl, Methoxy, Ethoxy, Trifluormethyl und/oder Trifluormethoxy substituiertes Phenyl, Phenoxy, Benzyl oder Benzyloxy und
- Z: für Sauerstoff, Schwefel oder eine Gruppierung der Formel -S(O)-, -SO₂-, -NH-, -N(CH₃)-, -CH₂O- oder -CH₂S- steht.
gefunden.

Die Verbindungen der Formel (I) können gegebenenfalls als geometrische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden daß man die neuen 3-Methoxy-2-phenyl-acrylsäuremethylester der allgemeinen Formel (I), 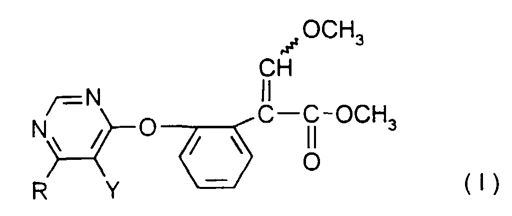 in welcher
- Y und R: die oben angegebenen Bedeutung haben
erhält, wenn man
a) 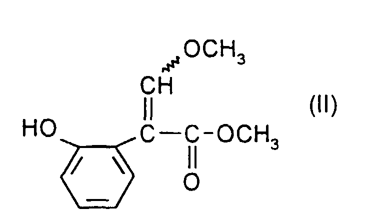 mit Pyrimidin-Derivaten der Formel (III) 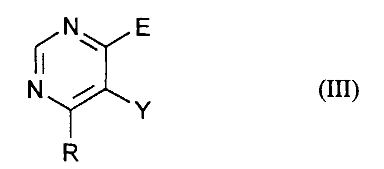 in welcher
   - R und Y: die oben angegebenen Bedeutung haben und
   - E: für eine elektronenanziehende Abgangsgruppe steht,
   gegebenenfalls in Gegenwart eines Verdünnungsmittels und gegebenenfalls in Gegenwart eines Reaktionshilfsmittels umsetzt;
   oder
b) die nach dem Verfahren a) erhaltenen 3-Methoxy-2-(halogenpyrimidinyloxyphenyl)-acrylsäuremethylester der Formel ( Ia) 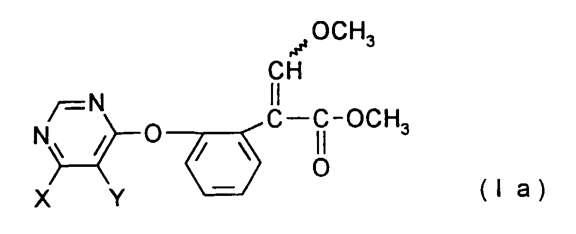 in welcher
   - X: für Fluor, Chlor, Brom oder Iod steht,
   - und Y: für Fluor oder Chlor steht
   mit Verbindungen der Formel ( IV )

   Ar-Z-H (IV)

   in welcher
   Ar und Z die oben angegebenen Bedeutung haben,
gegebenenfalls in Gegenwart eines Verdünnungsmittels und gegebenenfalls in Gegenwart eines Reaktionshilfsmittels sowie gegebenenfalls in Gegenwart eines Katalysators umsetzt.

Schließlich wurde gefunden, daß die neuen 3-Methoxy-2-phenyl-acrylsäuremethylester der allgemeinen Formel (I) gute Wirksamkeit gegenüber Schädlingen besitzen.

Überraschenderweise zeigen die erfindungsgemäßen 3-Methoxy-2-phenyl-acrylsäuremethylester der allgemeinen Formel (I) eine erheblich bessere Wirksamkeit gegenüber pflanzenschädigenden Mikroorganismen im Vergleich zu den aus dem Stand der Technik bekannten 3-Methoxy-acrylsäuremethylestern, wie beispielsweise die Verbindung 3-Methoxy-2-(6-phenyl-2-pyridylthio)-acrylsäuremethylester oder die Verbindung 3-Methoxy-2-[N-(6-phenyl-2-pyridyl)-N-methyl-amino]-acrylsäuremethylester oder die Verbindung 3-Methoxy-2-[5-(4-chlorphenyl)-3-pyridyloxy]-acrylsäuremethylester, welche chemisch und/oder wirkungsmäßig naheliegende Verbindungen sind.

Im einzelnen seien außer den bei den Herstellungsbeispielen genannten Verbindungen die folgenden 3-Methoxy-2-phenyl-acrylsäuremethylester der allgemeinen Formel (Ib) genannt:

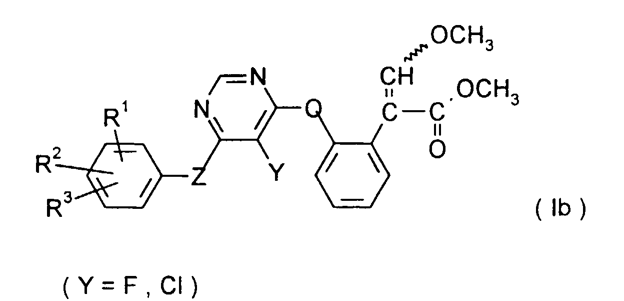

| **R**^{**1**} | **R**^{**2**} | **R**^{**3**} | **Z** |
|---|---|---|---|
| H | 2-CH₃ | 4-OCF₃ | -O- |
| H | 2-Cl | 4-OCF₃ | -O- |
| H | 2-CH₃ | 4-OCF₃ | -O- |
| H | 3,4-O-CF₂-CF₂-O- | | -O- |
| H | 3,4-O-CF₂-O- | | -O- |

Verwendet man beispielsweise 5-Chlor-4,6-difluorpyrimidin und 2-(2-Hydroxyphenyl)-3-methoxy-acrylsäuremethylester als Ausgangsstoffe, so läßt sich der Reaktionsablauf des erfingungsgemäßen Verfahrens ( a ) durch das folgende Formelschema darstellen:

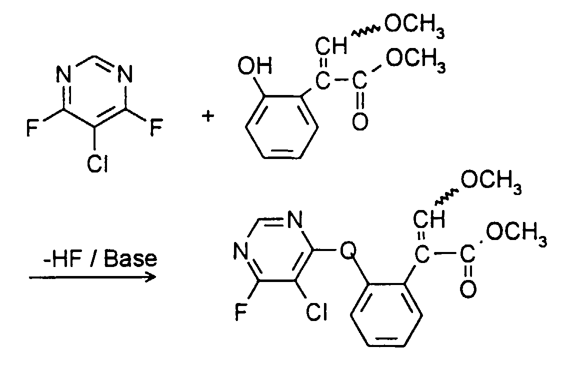

Verwendet man beispielsweise 2-[2-(5-Chlor-6-fluor-4-pyrimidinyloxy)-phenyl]-3-methoxy-acrylsäuremethylester und 2-Hydroxybenzonitril als Ausgangsstoffe, so läßt sich der Reaktionsablauf des erfindungsgemäßen Verfahrens ( b ) durch das folgende Formelschema darstellen:

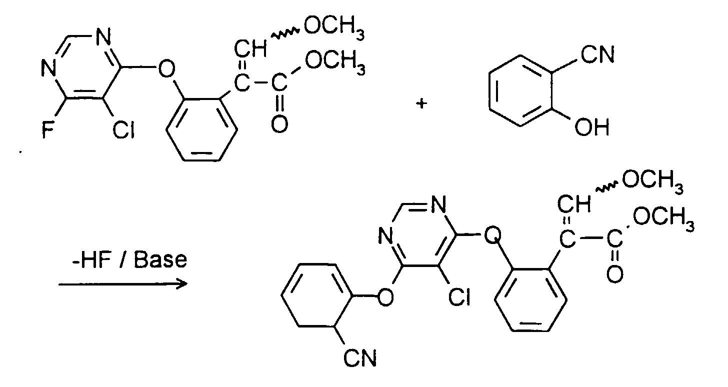

Der zur Durchführung des erfindungsgemäßen Verfahrens (a) als Ausgangsverbindung benötigte 2-Hydroxyphenyl-3-methoxyacrylsäureester ist durch die Formel (II) definiert. 2-Hydroxyphenyl-3-methoxyacrylsäureester der Formel (II) ist bekannt (vergl. z.B. EP 242 081).

Die zur Durchführung des erfindungsgemäßen Verfahrens (a) weiterhin als Ausgangsstoffe benötigten Pyrimidin-Derivate sind durch die Formel (III) allgemein definiert. In dieser Formel (III) stehen R und Y vorzugsweise für diejenigen Reste, die bereits im Zusammenhang mit der Beschreibung der erfindungsgemäßen Verbindungen der Formel (I) als bevorzugt für diese Substituenten genannt wurden. E steht für einen elektronenanziehenden Abgangsrest, vorzugsweise für Halogen, insbesondere für Fluor, Chlor, Brom oder Iod oder für jeweils gegebenenfalls substituiertes Alkylsulfonyloxy, Alkoxysulfonyloxy oder Arylsulfonyloxy, wie insbesondere Methansulfonyloxy, Trifluormethansulfonyloxy, Methoxysulfonyloxy, Ethoxysulfonyloxy oder p-Toluolsulfonyloxy.

Die Pyrimidin-Derivate der Formel (III) sind bekannt (vgl. z.B. DE-OS 1 931 640) bzw. nach in der Literatur beschriebenen Standardmethoden erhältlich.

Die zur Durchführung des erfindungsgemäßen Verfahrens (b) als Ausgangsstoffe benötigten3-Methoxy-2-(halogen-pyrimidinyloxy-phenyl)-acrylsäuremethylester sind durch die Formel (Ia) allgemein definiert. In dieser Formel (Ia) steht X für Fluor, Chlor, Brom oder Iod und Y für Fluor oder Chlor.

Die 3-Methoxy-2-(halogenpyrimidinyloxy-phenyl)-acrylsäuremethylester der Formel (Ia) sind erfindungsgemäße Verbindungen.

Die zur Durchführung des erfindungsgemäßen Verfahrens (b) weiterhin als Ausgangsstoffe benötigten Verbindungen sind durch die Formel (IV) allgemein definiert. In dieser Formel (IV) stehen Ar und Z vorzugsweise für diejenigen Reste, die bereits im Zusammenhang mit der Beschreibung der erfindungsgemäßen Verbindungen der Formel (I) als bevorzugt für diese Substituenten genannt wurden.

Die Verbindungen der Formel (IV) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid oder Sulfoxide, wie Dimethylsulfoxid.

Das erfindungsgemäße Verfahren (a) kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributylmethylphosphoniumbromid, Trimethyl-C₁₃/C₁₅-alkylammoniumchlorid, Trimethyl-C₁₃/C₁₅-alkylammoniumbromid, Dibenzyldimethylammoniummethylsulfat, Dimethyl-C₁₂/C₁₄-alkylbenzylammoniumchlorid, Dimethyl-C₁₂/C₁₄-alkylbenzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Das erfindungsgemäße Verfahren (a) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen -30°C und +120°C, vorzugsweise bei Temperaturen zwischen -20°C und +60°C.

Das erfindungsgemäße Verfahren (a) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (a) setzt man pro Mol an 2-Hydroxyphenyl-3-methoxyacrylsäuremethylester der Formel (II) im allgemeinen 1,0 bis 10,0 Mol, vorzugsweise 1,0 bis 5,0 Mol an Pyrimidin-Derivat der Formel (III) und gegebenenfalls 1,0 bis 5,0 Mol, vorzugsweise 1,0 bis 2,5 Mol an Reaktionshilfsmittel ein. Die Reaktionsdurchführung, Aufarbeitung und Isolierung der Reaktionsprodukte erfolgt nach bekannten Verfahren (vergl. hierzu auch die Herstellungsbeispiele).

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid oder Sulfoxide, wie Dimethylsulfoxid.

Das erfindungsgemäße Verfahren (b) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Das erfindungsgemäße Verfahren (b) wird außerdem gegebenenfalls in Gegenwart eines geeigneten Katalysators durchgeführt. Als solche kommen insbesondere Kupfer(I)-Salze, wie beispielsweise Kupfer-(I)-chlorid infrage. Hierbei kann der Zusatz von katalytischen Mengen eines geeigneten Phasentransferkatalysators, wie beispielsweise 15-Krone-5, 18-Krone-6 oder Tris-[2-(2-methoxyethoxy)-ethyl]-amin von Vorteil sein.

Die Reaktionstemperaturen können bei der Durchführung des erfindungsgemäßen Verfahrens (b) in einem größeren Bereich variiert werden. Im allgemeinen arbeitet man bei Temperaturen zwischen -20°C und +180°C, vorzugsweise bei Temperaturen zwischen 0°C und +150°C.

Das erfindungsgemäße Verfahren (b) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (b) setzt man pro Mol an 3-Methoxy-2-(halogenpyrimidinyloxy-phenyl)-acrylsäuremethylester der Formel (Ia) im allgemeinen 1,0 bis 3,0 Mol, vorzugsweise 1,0 bis 1,5 Mol an Verbindung der Formel (IV) und gegebenenfalls 0,1 bis 3,0 Mol, vorzugsweise 0,5 bis 1,5 Mol an als Reaktionshilfsmittel verwendeter Base ein. Die Reaktionsdurchführung, Aufarbeitung und Isolierung der Reaktionsprodukte erfolgt jeweils in Analogie zu bekannten Verfahren (vergl. hierzu beispielsweise EP 382 375 sowie die Herstellungsbeispiele).

Die Reinigung der Endprodukte der Formel (I) erfolgt mit Hilfe üblicher Verfahren, beispielsweise durch Säulenchromatographie oder durch Umkristallisieren.
Die Charakterisierung erfolgt mit Hilfe des Schmelzpunktes oder bei nicht kristallisierenden Verbindungen mit Hilfe des Brechungsindex oder der Protonen-Kernresonanzspektroskopie (¹H-NMR).

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei können die erfindungsgemäßen Wirkstoffe mit besonders gutem Erfolg zur Bekämpfung von Krankheiten im Obst- und Gemüseanbau, wie beispielsweise gegen den Erreger der Tomatenbraunfäule (Phytophthora infestans) oder gegen den Erreger des Apfelschorfes (Venturia inaequalis) oder zur Bekämpfung von Getreidekrankheiten wie beispielsweise gegen den Erreger des echten Getreidemehltaues (Erysiphe graminis) oder gegen den Erreger der Netzfleckenkrankheit der Gerste (Pyrenophora teres) oder gegen den Erreger der Braunspelzigkeit des Weizens (Leptosphaeria nodorum) oder gegen Fusariumarten an Getreide oder zur Bekämpfung von Reiskrankheiten, wie beispielsweise gegen den Erreger der Reisfleckenkrankheit (Pyricularia oryzae) eingesetzt werden. Daneben besitzen die erfindungsgemäßen Wirkstoffe eine gute in vitro-Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatsche Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen treten dabei synergistische Effekte auf.

Für die Mischungen kommen beispielsweise infrage:

### Fungizide:

2-Aminobutan;2-Anilino-4-methyl-6-cyclopropyl-pyrimidin;2',6'-Dibromo-2-methyl-4'-trifluoromethoxy-4'-trifluoro-methyl-1,3-thizole-5-carboxanilid, 2,6-Dichloro-N-(4-trifluoromethylbenzyl)benzamid;(E)-2-Methoxyimino-N-methyl-2-(2-phenoxyphenyl) acetamid; 8-Hydroxyquinolinsulfat; Methyl-(E)-2-{2-[6-(2-cyanophenoxy)pyrimidin-4-yloxy]phenyl}-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodin, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chloretoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin,
Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, ,Primiphos A, Profenofos, Profenophos, Promecarb, Propaphos, Propoxur, Prothiofos, Prothiophos, Prothoat, Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstöffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele

### Beispiel 1:

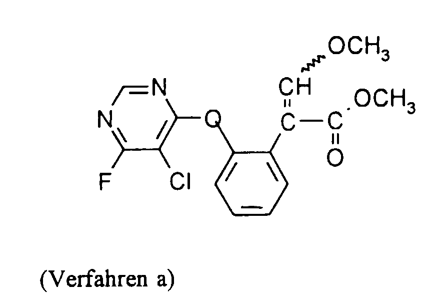

Zu einer Mischung aus 15 g (0,1 Mol) 5-Chlor-4,6-difluorpyrimidin und 20,8 g (0,1 Mol) 2-(2-Hydroxyphenyl)-3-methoxy-acrylsäuremethylester in 100 ml Dimethylformamid gibt man bei 0°C portionsweise 3 g (0,1 Mol) Natriumhydrid (80 %ig in Paraffinöl), läßt den Reaktionsansatz auf Raumtemperatur kommen und rührt anschließend 24 Stunden nach. Das Reaktionsgemisch wird anschließend auf Wasser gegeben, mit Essigsäureethylester extrahiert und im Vakuum eingeengt. Der Rückstand wird an Kieselgel chromatographiert (Laufmittel: Diethylether/Petrolether 1:1).

Man erhält 20,1 g (59,3 % der Theorie) 2-[2-(5-Chlor-6-fluor-4-pyrimidinyloxy)-phenyl]-3-methoxy-acrylsäuremethylester vom Schmelzpunkt 102°C.

### Beispiel 2:

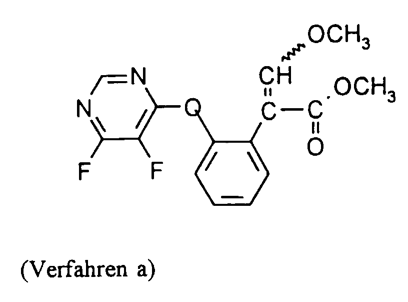

Zu einer Mischung aus 16,1 g (0,12 Mol) 4,5,6-Trifluorpyrimidin und 25,2 g (0,12 Mol) 2-(2-Hydroxyphenyl)-3-methoxy-acrylsäuremethylester in 120 ml Dimethylformamid gibt man bei 0°C portionsweise 3,6 g (0,12 Mol) Natriumhydrid (80 %ig in Paraffinöl), läßt den Reaktionsansatz auf Raumtemperatur kommen und rührt anschließend zwei Stunden nach. Das Reaktionsgemisch wird anschließend auf Wasser gegeben, mit Essigsäureethylester extrahiert und im Vakuum eingeengt. Der Rückstand wird an Kieselgel chromatographiert (Laufmittel: Diethylether/Petrolether 1:1.

Man erhält 3,6 g (9,6 % der Theorie) 2-[2-(5,6-Difluor-4-pyrimidinyloxy)-phenyl]-3-methyloxy-acrylsäuremethylester vom Schmelzpunkt 69°C.

### Beispiel 3:

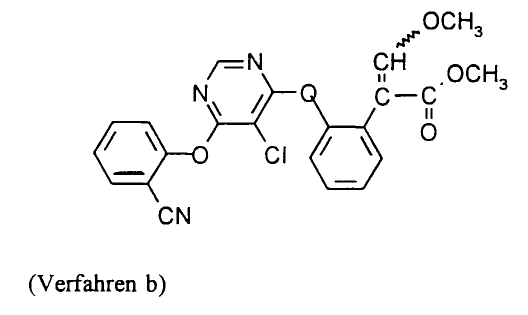

Zu einer Lösung von 8,46 g (0,025 Mol) 2-[2-(5-Chlor-6-fluor-4-pyrimidinyloxy)-phenyl]-3-methoxy-acrylsäuremethylester (Bsp. 1) und 3 g (0,025 Mol) 2-Hydroxybenzonitril in 50 ml Dimethylformamid gibt man bei 0°C portionsweise 0,75 g (0,025 Mol) Natriumhydrid (80 %ig in Paraffinöl) und rührt das Reaktionsgemisch 16 Stunden bei 25°C nach. Anschließend wird das Reaktionsgemisch auf Wasser gegeben, mit Essigsäureethylester extrahiert und im Vakuum eingeengt. Der Rückstand wird 10 Minuten in ter.-Butylmethylether unter Rückfluß erhitzt und filtriert.

Man erhält 8,9 g (81,4 % der Theorie) 2-{2-[5-Chlor-6-(2-cyanophenoxy)-4-pyrimidinyloxy]-phenyl}-3-methoxy-acrylsäuremethylester vom Schmelzpunkt 159°C - 61°C.

In entsprechender Weise und gemäß den allgemeinen Angaben zur Herstellung erhält man die folgenden 3-Methoxy-2-phenyl-acrylsäuremethylester der allgemeinen Formel (I):

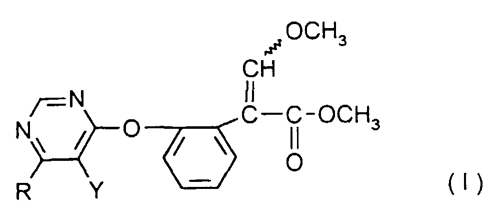

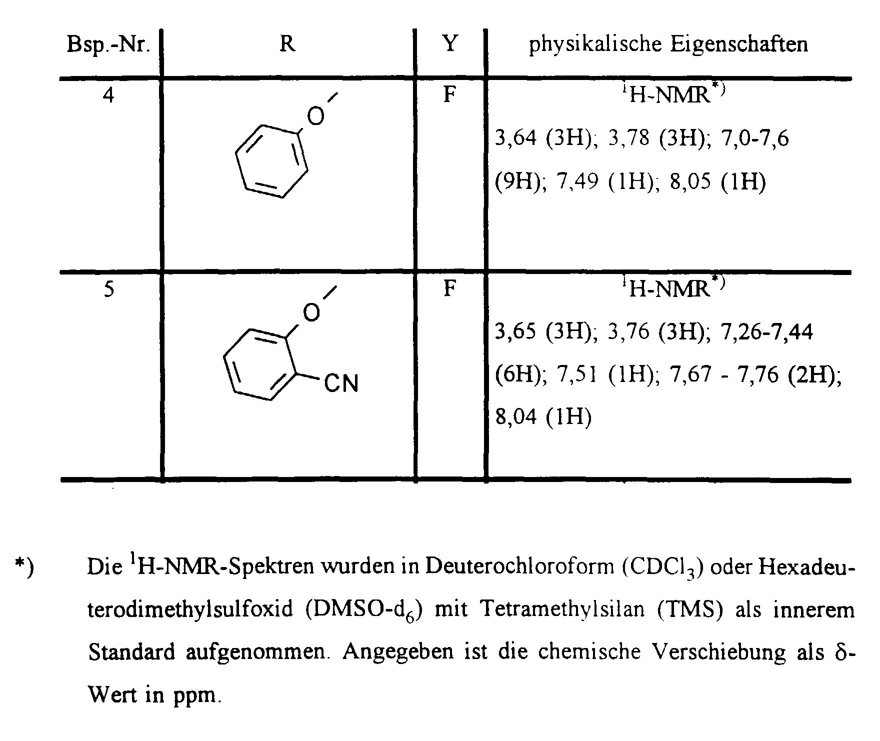

### Anwendungsbeispiele:

In den folgenden Anwendungsbeispielen wurden die nachstehend aufgeführten Verbindungen als Vergleichssubstanzen eingesetzt: 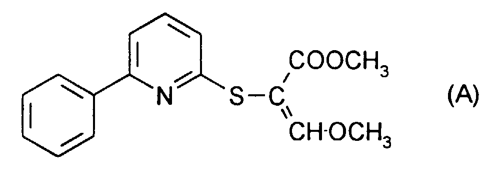 3-Methoxy-2-(6-phenyl-2-pyridylthio)-acrylsäuremethylester 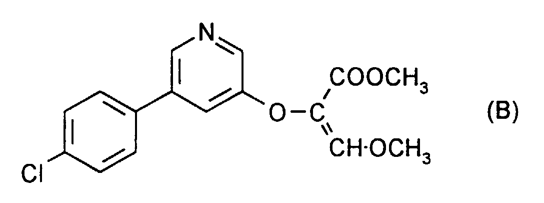 3-Methoxy-2-[5-(4-chlorphenyl)-3-pyridyloxy]-acrylsäuremethylester 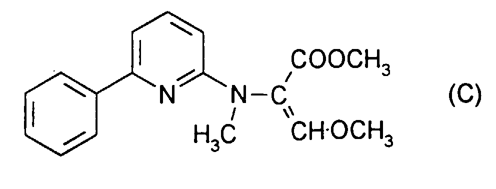 3-Methoxy-2-[N-(6-phenyl-2-pyridyl)-N-methyl-amino]-acrylsäuremethylester (alle bekannt aus EP 383 117).

### Beispiel A:

### Erysiphe-Test (Gerste) / protektiv

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der angegebenen Menge Wirkstoffzubereitung. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen 4 und 5, die bei einer Dosierung des Wirkstoffs von 400 g/ha einen über 80 %igen Wirkungsgrad zeigen.

### Beispiel B:

### Pyrenophora teres-Test (Gerste) / protektiv

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der angegebenen Menge Wirkstoffzubereitung. Nach Antrocknen des Spritzbelages werden die Pflanzen mit einer Konidiensuspension von Pyrenophora teres besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen 4 und 5, die bei einer Dosierung des Wirkstoffs von 400 g/ha einen bis zu 100 %igen Wirkungsgrad zeigen.

### Beispiel C:

### Phytophthora-Test (Tomate) / protektiv

| | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit von ca. 20 % aufgestellt.

3 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen 4 und 5, die bei einer Wirkstoffkonzentration von 10 ppm einen über 80 %igen Wirkungsgrad zeigen.

### Beispiel D:

### Venturia-Test (Apfel) / protektiv

| | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension des Apfelschorferregers Venturia inaequalis inokuliert und verbleiben dann 1 Tag bei 20°C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen 1, 3, 4, 5 und 6, die bei einer Wirkstoffkonzentration von 10 ppm einen bis zu 100 %igen Wirkungsgrad zeigen.

### Beispiel E:

### Pyricularia-Test (Reis) / protektiv

| | |
|---|---|
| Lösungsmittel | 12,5 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Reispflanzen mit der Wirkstoffzubereitung bis zur Tropfnässe. Nach Antrocknen des Spritzbelages werden die Pflanzen mit einer wäßrigen Sporensuspension von Pyricularia oryzae inokuliert. Anschließend werden die Pflanzen in einem Gewächshaus bei 100 % relativer Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen bei diesem Test z.B. die Verbindungen gemäß den Herstellungsbeispielen 4, 5 und 6, die bei einer Wirkstoffkonzentration von 0,025 % einen bis zu 100 %igen Wirkungsgrad zeigen.

## Patentansprüche

1. Verbindungen der Formel (I), 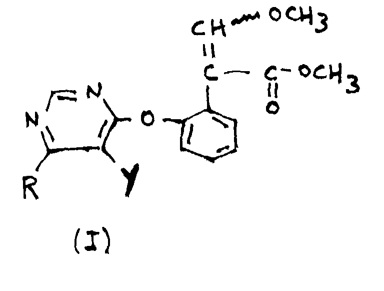 bei welchen
Y für Fluor oder Chlor steht,
R für Fluor, Chlor, Brom oder für einen Rest der Formel Ar-Z- steht, wobei
Ar für jeweils gegebenenfalls einfach bis dreifach, gleich oder verschieden substituiertes Phenyl oder Naphthyl oder für jeweils gegebenenfalls einfach bis dreifach, gleich oder verschieden substituiertes und/oder benzoannelliertes Furanyl, Thienyl, Pyrrolyl, Oxazolyl, Thiazolyl, Isoxazolyl, Isothiazolyl, Pyrazolyl, Imidazolyl, Oxadiazolyl, Thiadiazolyl, Triazolyl, Pyridyl, Pyridazinyl, Pyrimidinyl, Pyrazinyl oder Triazinyl steht, wobei als Substituenten jeweils infrage kommen:
Fluor, Chlor, Brom, Hydroxy, Cyano, Nitro, Amino, Formyl, Carbamoyl, Thiocarbamoyl, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Allyl, Butenyl, Allyloxy, Butenyloxy, Trifluormethyl, Trifluormethoxy, Trifluormethylthio, Trifluormethylsulfinyl, Trifluormethylsulfonyl, Dimethylamino, Diethylamino, Acetyl, Acetoxy, Methylsulfonyloxy, Ethylsulfonyloxy, Methoxycarbonyl, Ethoxycarbonyl, Hydroximinomethyl, Hydroximinoethyl, Methoximinomethyl, Methoximinoethyl, Ethoximinomethyl, Ethoximinoethyl, Propan-1,3-diyl, Butan-1,4-diyl, Dioxyethylen, Dioxyethylen, Dioxypropylen, Difluordioxymethylen, Tetrafluordioxyethylen, Cyclopropyl, Cyclopentyl, Cyclohexyl, 1-Pyrrolidinyl, 1-Piperidinyl, 1-Perhydroazepinyl, 4-Morpholinyl oder jeweils gegebenenfalls ein- bis dreifach, gleich oder verschieden durch Fluor, Chlor, Brom, Methyl, Ethyl, Methoxy, Ethoxy, Trifluormethyl und/oder Trifluormethoxy substituiertes Phenyl, Phenoxy, Benzyl oder Benzyloxy und
Z für Sauerstoff, Schwefel oder eine Gruppierung der Formel -S(O)-, -SO₂-, -NH-, -N(CH₃)-, -CH₂O- oder -CH₂S- steht.

2. Schädlingsbekämpfungsmittel, gekennzeichnet durch einen Gehalt an mindestens einer Verbindung der Formel (I) nach Anspruch 1.

3. Verfahren zur Bekämpfung von Pflanzenschädlingen, dadurch gekennzeichnet, daß man Verbindungen der Formel (I) nach Anspruch 1 auf Pflanzenschädlinge und/oder ihren Lebensraum einwirken läßt.

4. Verfahren zur Herstellung von 3-Methoxy-2-phenyl-acrylsäuremethylestern wie in Anspruch 1 definiert,
dadurch gekennzeichnet, daß man
a) 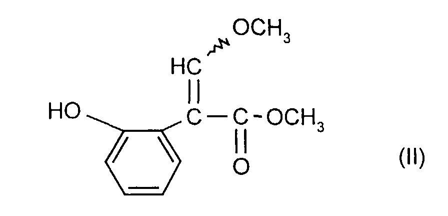 mit Pyrimidin-Derivaten der Formel (III) 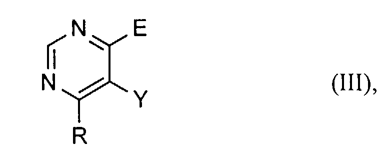 in welcher
R und Y die in Anspruch 1 angegebenen Bedeutung haben und
E für eine elektronenanziehende Abgangsgruppe steht,
gegebenenfalls in Gegenwart eines Verdünnungsmittels und gegebenenfalls in Gegenwart eines Reaktionshilfsmittels umsetzt;
oder
b)
die nach dem Verfahren a) erhaltenen 3-Methoxy-2-(halogenpyrimidinyloxyphenyl)-acrylsäuremethylester der Formel (Ia) 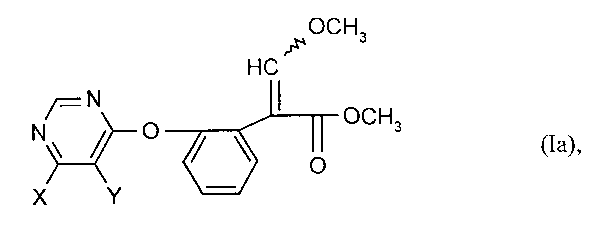 in welcher
X für Fluor, Chlor, Brom oder Iod stehen,
und Y für Fluor oder Chlor steht
mit Verbindungen der Formel (IV)
Ar-Z-H (IV)
in welcher
Ar und Z die in Anspruch 1 angegebenen Bedeutung haben
gegebenenfalls in Gegenwart eines Verdünnungsmittels und gegebenenfalls in Gegenwart eines Reaktionshilfsmittels sowie gegebenenfalls in Gegenwart eines Katalysators umsetzt.

5. Verwendung von Verbindungen der Formel (I) nach Anspruch 1 zur Bekämpfung von Schädlingen.

6. Verfahren zur Herstellung von Schädlingsbekämpfungsmitteln, dadurch gekennzeichnet, daß man Verbindungen der Formel (I) nach Anspruch 1 mit Streckmitteln und/oder oberflächenaktiven Mitteln vermischt.

## Claims

1. Compound of the formula (I) 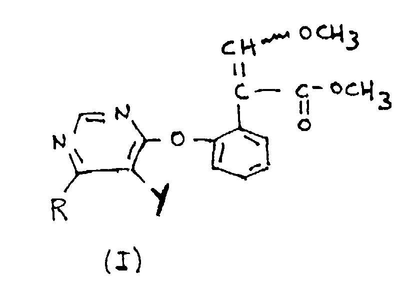 in which
Y represents fluorine or chlorine,
R represents fluorine, chlorine, bromine or a radical of the formula -Ar-Z- in which
Ar represents phenyl or naphthyl, each of which is optionally monosubstituted to trisubstituted by identical or different substituents, or represents furanyl, thienyl, pyrrolyl, oxazolyl, thiazolyl, isoxazolyl, isothiazolyl, pyrazolyl, imidazolyl, oxadiazolyl, thiadiazolyl, triazolyl, pyridyl, pyridazinyl, pyrimidinyl, pyrazinyl or triazinyl, each of which is optionally monosubstituted to trisubstituted by identical or different substituents and/or benzo-fused, suitable substituents in each case being:
fluorine, chlorine, bromine, hydroxyl, cyano, nitro, amino, formyl, carbamoyl, thiocarbamoyl, methyl, ethyl, n- or i-propyl, n-, i-, s- or t-butyl, methoxy, ethoxy, n- or i-propoxy, n-, i-, s- or t-butoxy, methylthio, ethylthio, methylsulphinyl, methylsulphonyl, allyl, butenyl, allyloxy, butenyloxy, trifluoromethyl, trifluoromethoxy, trifluoromethylthio, trifluoromethylsulphinyl, trifluoromethylsulphonyl, dimethylamino, diethylamino acetyl, acetoxy, methylsulphonyloxy, ethylsulphonyloxy, methoxycarbonyl, ethoxycarbonyl, hydroximinomethyl, hydroximinoethyl, methoximinomethyl, methoximinoethyl, ethoximinomethyl, ethoximinoethyl, propane-1,3-diyl, butane-1,4-diyl, dioxyethylene, dioxyethylene, dioxypropylene, difluorodioxymethylene, tetra-fluorodioxyethylene, cyclopropyl, cyclopentyl, cyclohexyl, 1-pyrrolidinyl, 1-piperidinyl, 1-perhydroazepinyl, 4-morpholinyl, or phenyl, phenoxy, benzyl or benzyloxy, each of which is optionally monosubstituted to trisubstituted by identical or different substituents from the series comprising fluorine, chlorine, bromine, methyl, ethyl, methoxy, ethoxy, trifluoromethyl and/or trifluoromethoxy, and
z represents oxygen, sulphur or a group of the formula -S(O)-, -SO₂-, -NH-, -N(CH₃)-, -CH₂O- or -CH₂S-.

2. Pesticide, characterized by a content of at least one compound of the formula (I) according to Claim 1.

3. Method of combating plant pests, characterized in that compounds of the formula (I) according to Claim 1 are allowed to act on plant pests and/or their environment.

4. Process for the preparation of methyl 3-methoxy-2-phenyl-acrylates as defined in Claim 1,
characterized in that
a) 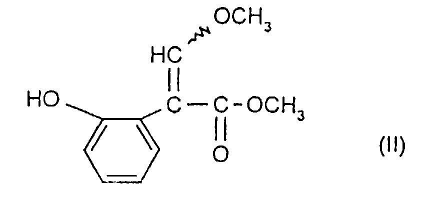 is reacted with pyrimidine derivatives of the formula (III) 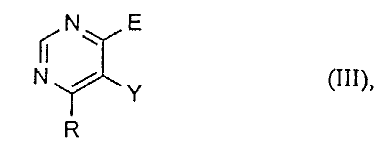 in which
R and Y have the abovementioned meaning and
E represents an electron-attracting leaving group,
if appropriate in the presence of a diluent and if appropriate in the presence of a reaction auxiliary;
or
b) the methyl 3-methoxy-2-(halogenopyrimidinyloxyphenyl)-acrylates obtained by process a), of the formula (Ia) 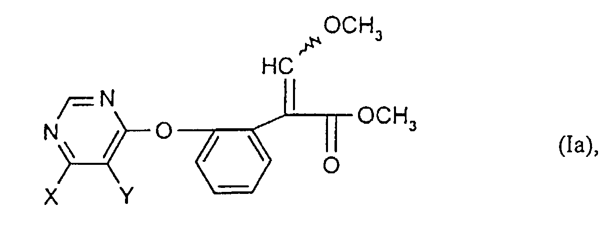 in which
X represents fluorine, chlorine, bromine or iodine,
and Y represents fluorine or chlorine,
are reacted with compounds of the formula (IV)
Ar-Z-H (IV)
in which
Ar and Z have the abovementioned meaning
if appropriate in the presence of a diluent and if appropriate in the presence of a reaction auxiliary and if appropriate in the presence of a catalyst.

5. Use of compounds of the formula (I) according to Claim 1 for combating pests.

6. Process for the preparation of pesticides, characterized in that compounds of the formula (I) according to Claim 1 are mixed with extenders and/or surface-active agents.

## Revendications

1. Composés de formule (I) 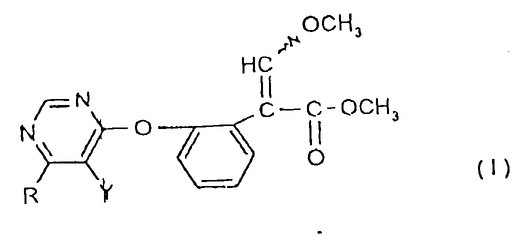 dans lesquels
Y représente le fluor ou le chlore,
R représente le fluor, le chlore, le brome ou un reste de formule Ar-Z, dans laquelle
Ar désigne un groupe, éventuellement substitué une à trois fois identiques ou différentes, phényle ou naphtyle ou un groupe, éventuellement substitué une à trois fois identiques ou différentes et/ou condensé au benzène, furannyle, thiényle, pyrrolyle, oxazolyle, thiazolyle, isoxazolyle, isothiazolyle, pyrazolyle, imidazolyle, oxadiazolyle, thiadiazolyle, triazolyle, pyridyle, pyridazinyle, pyrimidinyle, pyrazinyle ou triazinyle, en considérant dans chaque cas comme substituants :
fluor, chlore, brome, hydroxy, cyano, nitro, amino, formyle, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, méthylthio, éthylthio, méthylsulfinyle, méthylsulfonyle, allyle, butényle, allyloxy, butényloxy, trifluorométhyle, trifluorométhoxy, trifluorométhylthio, trifluorométhylsulfinyle, trifluorométhylsulfonyle, diméthylamino, diéthylamino, acétyle, acétoxy, méthylsulfonyloxy, éthylsulfonyloxy, méthoxycarbonyle, éthoxycarbonyle, hydroximinométhyle, hydroximinoéthyle, méthoximinométhyle, méthoximinoéthyle, éthoximinométhyle, éthoximinoéthyle, propane-1,3-diyle, butane-1,4-diyle, dioxyéthylène, dioxypropylène, difluorodioxyméthylène, tétrafluorodioxyéthylène, cyclopropyle, cyclopentyle, cyclohexyle, 1-pyrrolidinyle, 1-pipéridinyle, 1-perhydroazépinyle, 4-morpholinyle ou bien un groupe phényle, phénoxy, benzyle ou benzyloxy portant chacun le cas échéant un à trois substituants, identiques ou différents, fluoro, chloro, bromo, méthyle, éthyle, méthoxy, éthoxy, trifluorométhyle et/ou trifluoro-méthoxy et
Z représente l'oxygène, le soufre ou un groupement de formule -S(O)-, -SO₂-, -NH-, -N(CH₃)-, -CH₂O- ou -CH₂S-.

2. Compositions pesticides, caractérisées par une teneur en au moins un composé de formule (I) suivant la revendication 1

3. Procédé pour combattre des parasites des plantes,
caractérisé en ce qu'on fait agir des composés de formule (I) suivant la revendication 1 sur des parasites des plantes et/ou sur leur milieu.

4. Procédé de production d'esters méthyliques d'acides 3-méthoxy-2-acryliques tels que définis dans la revendication 1,
caractérisé en ce qu'on fait réagir
a) 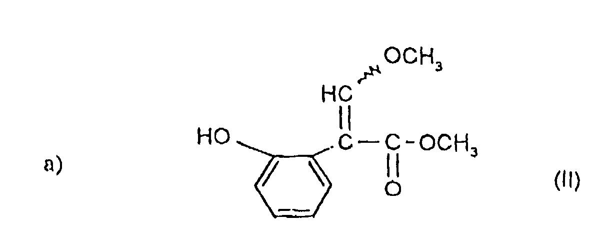 avec des dérivés de pyrimidine de formule (III) 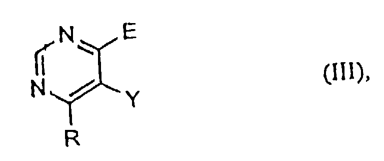 dans laquelle
R et Y ont la définition indiquée ci-dessus et
E représente un groupe partant attirant les électrons,
le cas échéant en présence d'un diluant et en présence éventuelle d'un auxiliaire de réaction ;
ou bien
b)
on fait réagir les esters méthyliques d'acides 3-méthoxy-2-(halogénopyrimidinyloxyphényl)-acryliques obtenus selon le procédé a), de formule (Ia) 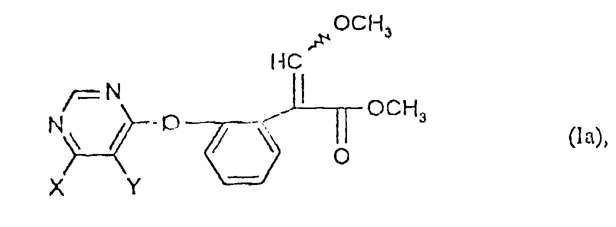 dans laquelle
x représente le fluor, le chlore, le brome ou l'iode et
Y représente le fluor ou le chlore,
avec des composés de formule (IV)
Ar-Z-H (IV)
dans laquelle
Ar et Z ont la définition indiquée ci-dessus,
le cas échéant en présence d'un diluant et en présence éventuelle d'un auxiliaire de réaction ainsi que, le cas échéant, en présence d'un catalyseur.

5. Utilisation de composés de formule (I) suivant la revendication 1 pour combattre des parasites.

6. Procédé de préparation de compositions pesticides, caractérisé en ce qu'on mélange des composés de formule (I) suivant la revendication 1 avec des diluants et/ou des agents tensio-actifs.
